# EUROPEAN PATENT APPLICATION

(11) **EP 1 444 978 A1**
(43) Date of publication of application: **11.08.2004**
(21) Application number: 02778095.6
(22) Date of filing: 12.11.2002
(51) Int. Cl.: A61K 31/198, A61K 9/20, A61K 47/10, A61K 47/26, A61P 3/02

(54) **AMINO ACID-CONTAINING TABLETS QUICKLY DISINTEGRATING IN THE ORAL CAVITY AND PROCESS FOR PRODUCING THE SAME**

(30) Priority: 13.11.2001 JP 2001347304
(71) Applicant: KYOWA HAKKO KOGYO CO., LTD., Chiyoda-ku, Tokyo 100-8185 (JP)
(72) Inventor: OHTA, Motohiro, c/o KYOWA HAKKO KOGYO CO., LTD, Sunto-gun, Shizuoka 411-8731 (JP); YOSHIMOTO, Hirokazu, c/o KYOWA HAKKO KOGYO CO. LTD, Sunto-gun, Shizuoka 411-8731 (JP); SAITO, Naohiro, c/o KYOWA HAKKO KOGYO CO., LTD, Sunto-gun, Shizuoka 411-8731 (JP); WATANABE, Yasushi, c/o KYOWA HAKKO KOGYO CO., LTD, Sunto-gun, Shizuoka 411-8731 (JP); MORIMOTO, Kiyoshi, c/o KYOWA HAKKO KOGYO CO., LTD, Sunto-gun, Shizuoka 411-8731 (JP)
(74) Representative: Teipel, Stephan, Dr.
(86) International application number: PCT/JP2002/011769
(87) International publication number: WO 2003/041698

(57) **Abstract**

There are provided an intraorally rapidly disintegrable tablet containing a large quantity of an amino acid and a method for manufacturing the same.

The intraorally rapidly disintegrable tablet is obtained by a manner wherein a mixture containing an amino acid and a disintegrating agent is granulated with an aqueous solution of a binder and dried, and the resulting granules are subjected to compression molding.

## Description

### Technical Field

The present invention relates to an intraorally rapidly disintegrable tablet containing a large quantity of an amino acid and also to a method for manufacturing the same.

### Background of the Invention

With regard to a health food or a dietary supplement containing an amino acid as a main ingredient, a sports drink where an amino acid is dissolved in water, a jelly containing an amino acid, granules mixed with an amino acid which are to be taken with water, etc. are currently known.

In the Field of pharmaceuticals, with regard to an intraorally rapidly disintegrable agent containing an amino acid, there have been already proposed oral tablets comprising water-soluble azulene having a stomachic effect, L-glutamine, disintegrating agent, a metal carbonate and an organic acid (Japanese Patent Laid-Open No. 2000-26286).

However, the intraorally rapidly disintegrable tablets containing glutamine disclosed in the Japanese Patent Laid-Open No. 2000-28286 are the so-called effervescent tablets where metal carbonate is decomposed upon contact to saliva in the mouth cavity to generate carbon dioxide gas (CO₂ gas) whereby disintegration of tablets is promoted and, therefore, they exhibit stimulation in the mouth cavity. In addition, a basic metal carbonate and an acidic organic acid are added to the intraorally rapidly disintegrable tablets containing glutamine which are disclosed in the Japanese Patent Laid-Open No. 2000-26286 and, therefore, there is a problem in terms of stability of an amino acid and there is further a problem that adjustment of the taste is hardly carried out. Accordingly, it cannot be said that the intraorally rapidly disintegrable tablets of such an effervescent type are preferable as intraorally rapidly disintegrable tablets to be used as a nutritious food.

Moreover, the intraorally rapidly disintegrable tablets containing glutamine disclosed in the Japanese Patent Laid-Open No. 2000-26286 are manufactured by the so-called inner lubrication method which is characterized in that a lubricant such as magnesium stearate or sodium laurylsulfate, talc, besides the effective ingredient and an excipitent, is kneaded with the materials for molding and tableting, followed by subjecting the mixture to compression molding. Thus, substantially, the content of the effective ingredient (main drug) contained in one tablet is small and, therefore, it is necessary to be administered plenty of tablets for taking a large quantity of an amino acid which is needed immediately after physical exercise.

On the other hand, there are disadvantages in a sports drink such as inconvenience for carrying, the sense of distension after drinking due to taking of a large quantity of water, and excessive excretion of sweat and urine. In the cases of a jelly and granules, they are difficult to take in during physical exercises. Even when they are made into tablets, it is necessary for the conventional oral tablets to be taken with water and, since it is difficult to take the tablets during the physical exercises such as jogging, they are not convenient as a health food for sports. In addition, it is necessary to subject the molding materials to compression molding with a considerable pressure, which causes a problem in the stability of a part of amino acids such as glutamine.

In order to solve the above-mentioned problems, there has been a demand for a development of an intraorally rapidly disintegrable tablet which contains a large amount of an amino acid as a principal agent of the nutritional composition and is disintegrated quickly in the mouth cavity.

### Disclosure of the Invention

An object of the present invention is to provide an intraorally rapidly disintegrable tablet containing an amino acid as a principal agent which quickly disintegrates upon contact to saliva in the mouth cavity and which can be taken without water, and also to provide a method for manufacturing the same.

Another object of the present invention is to provide an intraorally rapidly disintegrable tablet in which various kinds of amino acids are uniformly dispersed, and also to provide a method for manufacturing the same.

Still another object of the present invention is to provide an intraorally rapidly disintegrable tablet containing an amino acid, which is excellent in stability, as a principal agent, and also to provide a method for manufacturing the same.

Further object of the present invention is to provide an intraorally rapidly disintegrable tablet containing an amino acid as a principal agent and having a short disintegrating time in the mouth cavity, and also to provide a method for manufacturing the same.

Still further object of the present invention is to provide an intraorally rapidly disintegrable tablet containing an amino acid as a principal agent which hardly cracks even by a physical force caused during preservation and transportation, and also to provide a method for manufacturing the same.

Still further object of the present invention is to provide an intraorally rapidly disintegrable tablet containing an amino acid as a principal agent which contains no effervescent component and hardly stimulates the mouth cavity, and also to provide a method for manufacturing the same.

An intraorally rapidly disintegrable tablet as mentioned in claim 1 comprises a saccharide selected from the group consisting of lactose, maltose, trehalose, sorbitol, lactitol, mannitol, erythritol, xylitoland maltitol, one member selected from the group consisting of an amino acid and an amino acid derivative, and a disintegrating agent.

An intraorally rapidly disintegrable tablet as mentioned in claim 2 is manufactured by granulating a mixture containing one member selected from the group consisting of an amino acid and an amino acid derivative and a disintegrating agent with an aqueous solution of a binder to prepare granules, drying the granules, and subjecting the resulting granules to compression molding.

An intraorally rapidly disintegrable tablet as mentioned in claim 3 is manufactured by granulating a mixture containing one member selected from the group consisting of an amino acid and an amino acid derivative and a disintegrating agent with an aqueous solution of a binder to prepare granules, drying the granules, and subjecting a molding material comprising a mixture of the resulting granule and an additive to compression molding.

The intraorally rapidly disintegrable tablet as mentioned in claim 4 is the intraorally rapidly disintegrable tablet according to claim 2 or claim 3, wherein the aqueous solution of a binder is an aqueous solution of lactose.

The intraorally rapidly disintegrable tablet as mentioned in claim 5 is the intraorally rapidly disintegrable tablet according to claim 2 or claim 3, wherein the aqueous solution of a binder is an aqueous solution selected from the group consisting of an aqueous solution of maltose, an aqueous solution of trehalose, an aqueous solution of sorbitol, an aqueous solution of erythritol, an aqueous solution of xylitol, an aqueous solution of lactitol, an aqueous solution of mannitol and an aqueous solution of maltitol.

The intraorally rapidly disintegrable tablet as mentioned in claim 6 is the intraorally rapidly disintegrable tablet according to any of claims 3 to 5, wherein the additive contains an excipient selected from the group consisting of trehalose, mannitol, sorbitol, erythritol, lactitol, xylitol, maltitol and lactose.

The intraorally rapidly disintegrable tablet as mentioned in claim 7 is the intraorally rapidly disintegrable tablet according to any of claims 1 to 6, wherein the tablet is an externally lubricated tablet.

The intraorally rapidly disintegrable tablet as mentioned in claim 8 is the intraorally rapidly disintegrable tablet according to any of claims 1 to 7, wherein the tablet is a pharmaceutical preparation, a nutritious food or a dietary supplement.

A method for the manufacture of an intraorally rapidly disintegrable tablet mentioned in claim 9 comprises the steps of preparing a mixture containing a saccharide compound selected from the group consisting of lactose, maltose, trehalose, sorbitol, erythritol, lactitol, mannitol, xylitol and maltitol, an amino acid and a disintegrating agent, and subjecting the mixture to compression molding.

A method for the manufacture of an intraorally rapidly disintegrable tablet mentioned in claim 10 comprises the steps of: preparing a mixture containing one member selected from the group consisting of an amino acid and an amino acid derivative and a disintegrating agent, mixing the mixture with an aqueous solution of a binder to prepare granules; drying the granules, and subjecting the granules to compression molding.

A method for the manufacture of an intraorally rapidly disintegrable tablet mentioned in claim 11 comprises the steps of: preparing a mixture containing one member selected from the group consisting of an amino acid and an amino acid derivative and a disintegrating agent, mixing the mixture with an aqueous solution of a binder to prepare granules; drying the granules, mixing the granules with an additive to prepare a molding materials, and subjecting the molding material to compression molding.

A method for the manufacture of an intraorally rapidly disintegrable tablet mentioned in claim 12 is the method for the manufacture of the intraorally rapidly disintegrable tablet mentioned in claim 10 or claim 11 wherein the aqueous solution of a binder used is an aqueous solution of lactose.

A method for the manufacture of an intraorally rapidly disintegrable tablet mentioned in claim 13 is the method for the manufacture of the intraorally rapidly disintegrable tablet mentioned in claim 10 or claim 11, wherein the aqueous solution of a binder used is an aqueous solution selected from the group consisting of an aqueous solution of maltose, an aqueous solution of trehalose, an aqueous solution of sorbitol, an aqueous solution of erythritol, an aqueous solution of xylitol, an aqueous solution of lactitol, an aqueous solution of mannitol and an aqueous solution of maltitol.

A method for the manufacture of an intraorally rapidly disintegrable tablet mentioned in claim 14 is the method for the manufacture of the intraorally rapidly disintegrable tablet mentioned in any of claims 11 to 13, wherein the additive used contains an excipient selected from the group consisting of trehalose, mannitol, erythritol, lactitol, maltitol, xylitol, sorbitol, and lactose.

A method for the manufacture of an intraorally rapidly disintegrable tablet mentioned in claim 15 is the method for the manufacture of the intraorally rapidly disintegrable tablet mentioned in any of claims 9 to 14, wherein the step of compression molding is characterized in using a punch and a die to which a lubricant is applied.

A method for the manufacture of an intraorally rapidly disintegrable tablet mentioned in claim 16 is the method for the manufacture of the intraorally rapidly disintegrable tablet mentioned in any of claims 9 to 15, wherein the intraorally rapidly disintegrable tablet manufactured is a pharmaceutical preparation, a nutritious food or a dietary supplement.

An intraorally rapidly disintegrable tablet mentioned in claim 17 contains a saccharide selected from the group consisting of lactose, maltose, trehalose, sorbitol and maltitol, one member selected from the group consisting of an amino acid and an amino acid derivative and a disintegrating agent.

An intraorally rapidly disintegrable tablet as mentioned in claim 18 is manufactured by granulating a mixture containing one member selected from the group consisting of an amino acid and an amino acid derivative and a disintegrating agent with an aqueous solution of a binder to prepare granules, drying the granules, and subjecting the resulting granules to compression molding.

An intraorally rapidly disintegrable tablet as mentioned in claim 19 is manufactured by granulating a mixture containing one member selected from the group consisting of an amino acid and an amino acid derivative and a disintegrating agent with an aqueous solution of a binder to prepare granules, drying the granules, and subjecting a molding material comprising a mixture of the resulting granule and an additive to compression molding.

The intraorally rapidly disintegrable tablet as mentioned in claim 20 is the intraorally rapidly disintegrable tablet according to claim 18 or claim 19, wherein the aqueous solution of a binder is an aqueous solution of lactose.

The intraorally rapidly disintegrable tablet as mentioned in claim 21 is the intraorally rapidly disintegrable tablet according to claim 18 or claim 19, wherein the aqueous solution of a binder is an aqueous solution selected from the group consisting of an aqueous solution of maltose, an aqueous solution of trehalose, an aqueous solution of sorbitol and an aqueous solution of maltitol.

The intraorally rapidly disintegrable tablet as mentioned in claim 22 is the intraorally rapidly disintegrable tablet according to any of claims 19 to 21, wherein the additive contains an excipient selected from the group consisting of trehalose, mannitol and lactose.

The intraorally rapidly disintegrable tablet as mentioned in claim 23 is the intraorally rapidly disintegrable tablet according to any of claims 17 to 22, wherein the tablet is an externally lubricated tablet.

The intraorally rapidly disintegrable tablet as mentioned in claim 24 is the intraorally rapidly disintegrable tablet according to any of claims 17 to 23, wherein the tablet is a pharmaceutical preparation, a nutritious food or a dietary supplement.

A method for the manufacture of an intraorally rapidly disintegrable tablet mentioned in claim 25 comprises the step of preparing a mixture containing a saccharide selected from the group consisting of lactose, maltose, trehalose, sorbitol and maltitol, one member selected from the group consisting of an amino acid and an amino acid derivative and a disintegrating agent, and subjecting the mixture to compression molding.

A method for the manufacture of an intraorally rapidly disintegrable tablet mentioned in claim 26 comprises the steps of: preparing a mixture containing one member selected from the group consisting of an amino acid and an amino acid derivative and a disintegrating agent, mixing the mixture with an aqueous solution of a binder to prepare granules; drying the granules, and subjecting the granules to compression molding.

A method for the manufacture of an intraorally rapidly disintegrable tablet mentioned in claim 27 comprises the steps of: preparing a mixture containing one member selected from the group consisting of an amino acid and an amino acid derivative and a disintegrating agent, mixing the mixture with an aqueous solution of a binder to prepare a granules; drying the granules, mixing the granules with an additive to prepare a molding material, and subjecting the molding material to compression molding.

A method for the manufacture of an intraorally rapidly disintegrable tablet mentioned in claim 28 is the method for the manufacture of the intraorally rapidly disintegrable tablet mentioned in claim 26 or claim 27, wherein the aqueous solution of a binder used is an aqueous solution of lactose.

A method for the manufacture of an intraorally rapidly disintegrable tablet mentioned in claim 29 is the method for the manufacture of the intraorally rapidly disintegrable tablet mentioned in claim 26 or claim 27, wherein the aqueous solution of a binder used is an aqueous solution selected from the group consisting of an aqueous solution of maltose, an aqueous solution of trehalose, an aqueous solution of sorbitol and an aqueous solution of maltitol.

A method for the manufacture of an intraorally rapidly disintegrable tablet mentioned in claim 30 is the method for the manufacture of an intraorally rapidly disintegrable tablet mentioned in any of claims 27 to 29, wherein the additive used contains an excipient selected from the group consisting of trehalose, mannitol and lactose.

A method for the manufacture of an intraorally rapidly disintegrable tablet mentioned in claim 31 is the method for the manufacture of an intraorally rapidly disintegrable tablet mentioned in any of claims 25 to 30, wherein the step of compression molding is characterized in using a punch and a die to which a lubricant is applied.

A method for the manufacture of an intraorally rapidly disintegrable tablet mentioned in claim 32 is the method for the manufacture of an intraorally rapidly disintegrable tablet mentioned in any of claims 25 to 31, wherein the intraorally rapidly disintegrable tablet manufactured is a pharmaceutical preparation, a nutritious food or a dietary supplement.

### Best Mode for Carrying Out the Invention

Examples of the amino acid used in the present invention are aliphatic amino acids such as glycine and alanine; branched-chain amino acids such as valine, leucine and isoleucine; hydroxyamino acids such as serine and threonine; acidic amino acids such as aspartic acid and glutamic acid; amides such as asparagine and glutamine; basic amino acids such as lysine, hydroxylysine, arginine and ornithine; sulfur-containing amino acids such as cysteine, cystine and methionine; aromatic amino acids such as phenylalanine and tyrosine; heterocyclic amino acids such as tryptophane and histidine; or imino acids such as proline and 4-hydroxyproline.

Examples of the amino acid derivative are acetylglutamine, acetylcysteine, carboxymethylcysteine, acetyltyrosine, acetylhydroxyproline, 5-hydroxyproline, glutathione, creatine, S-adenylmethionine, glycylglycine, glycylglutamine, DOPA, alanylglutamine, carnitine, etc.

With regard to the amino acid used in the present invention, preferred are branched-chain amino acids such as valine, leucine and isoleucine, amides such as asparagine and glutamine, or basic amino acids such as lysine, hydroxylysine, arginine and ornithine; more preferred is branched-chain amino acids such as valine, leucine and isoleucine, or amides such as asparagine and glutamine; and particularly preferred are valine, leucine, isoleucine, glutamine and arginine.

Branched-chain amino acids such as valine, leucine and isoleucine, or amides such as asparagine and glutamine have a solubility of 0.5% to 10% in water of 25°C.

The intraorally rapidly disintegrable tablet according to the present invention may contain the amino acid or the amino acid derivative either solely or in combination. There is no particular limitation for the combination of amino acid or amino acid derivative, and any of two or more members selected from the group consisting of the above-mentioned amino acid and the above-mentioned amino acid derivative may be used in combination.

With regard to the combination of amino acid, a combination of two or more amino acids selected from the group consisting of a branched-chain amino acid, an amide and a basic amino acid are preferred.

Examples of the suitable combination of an amino acid are a combination of three kinds of branched-chain amino acids comprising valine, leucine and isoleucine; a combination of glutamine with one or more branched-chain amino acid(s); a combination of arginine with one or more branched-chain amino acid(s); a combination of glutamine and arginine with one or more branched-chain amino acid(s), and the like. Such a combination may be further combined with other kinds of an amino acid.

Although there is no particular limitation for the content of the amino acid in an intraorally rapidly disintegrable tablet, it is preferably 20 to 98% by weight, more preferably 35 to 95% by weight and, particularly preferably 50 to 93% by weight.

With regard to the disintegrating agent, preferred ones are crospovidone, croscarmellose sodium, low substituted hydroxypropylcellulose, carboxymethylcellulose, carboxymethylcellulose calcium and sodium carboxymethyl starch.

It is also possible to use a tablet containing 5 to 15% of water-absorbing amino acids such as proline, arginine, ornithine, lysine and glycine in the tablet as a disintegrating agent.

The disintegrating agent is mixed in the manufactured intraorally rapidly disintegrable tablet in an amount of 1 to 30% by weight, preferably 1 to 15% by weight, and more preferably 1 to 10% by weight.

The tablet of the present invention may contain two or more disintegrating agents.

As an aqueous solution of a binder, there is no particular limitation, and water such as pure water may be used solely, and preferably used is an aqueous solution containing a saccharide. There is no particular limitation for the saccharide, and its examples are lactose, maltose, trehalose, sorbitol, lactitol, mannitol, erythritol, xylitol or maltitol.

Amount of lactose, maltose, trehalose, sorbitol, lactitol, mannitol, erythritol, xylitol or maltitol used in the present invention as a binder in one intraorally rapidly disintegrable tablet is preferably 1 to 30% by weight, more preferably 1 to 20% by weight and, particularly preferably 1 to 10% by weight.

Although such a saccharide may be added in a form of powder, it is preferred that a saccharide is dissolved in water to prepare an aqueous solution containing the saccharide, which may be added to the tablet as a binder.

Although there is no particular limitation for the concentration of the saccharide in an aqueous solution of a binder, it is preferably 0.5 to 20% by weight, more preferably 2 to 15% by weight, and particularly preferably 5 to 10% by weight.

Lactose *per se* is nutritious as a carbohydrate, and it also has a property of being dissolved in water and having a low viscosity even after dissolved in water.

Also, an additive may be added to the intraorally rapidly disintegrable tablet. The additive may be added in any stage of manufacturing process of the intraorally rapidly disintegrable tablet of the present invention. For example, it may be mixed with a mixture containing an amino acid and a disintegrating agent or it may be mixed with granules prepared by granulation of a mixture containing an amino acid and a disintegrating agent.

As the additive, there is no particular limitation so far as it is a component which is able to be added to a pharmaceutical preparation, a nutritious food and a dietary supplement, and its examples are a nutritious carbohydrate, a coating agent, a flavor, sweetener, a corrigent, a coloring agent, a fluidizing agent, a lubricant and an excipient. Although there is no particular limitation for their concentration in the intraorally rapidly disintegrable tablet, it is preferably from a very small quantity to 20% by weight for each and particularly preferably from 0.1 to 10% by weight for each.

Examples of the nutritious carbohydrate are oligosaccharides such as reducing maltose which is a low-caloric saccharide and dextrin; cyclodextrin which is a corrigent for bitter taste; food dyes such as beta-carotene, ; niacin; vitamin E; ascorbic acid; vitamin B substances such as vitamin B₁, vitamin B₂, vitamin B₆ and vitamin B₁₂; vitamins such as vitamin A and vitamin D; minerals such as sodium; nicotinic acid amide; calcium pantothenate; folic acid, and the like.

Among the above-mentioned amino acids, leucine, isoleucine, valine, arginine, etc. have bitter taste. When such amino acid having bitter taste is contained in the intraorally rapidly disintegrable tablet, the surface may be coated with a coating agent such as that of a cellulosic type (e.g., hydroxypropylmethyl cellulose phthalate (HPMCP)), an acrylate type (e.g., shellac) or a natural type (e.g., methacrylic acid copolymer) so that the bitter taste is masked.

As the flavor, those which are commonly used in pharmaceuticals and foods such as flavor of a citrus type and menthol, may be used. As the sweetener and the corrigent, there may be exemplified sucralose, aspartame, glucose, fructose, saccharin, etc.

As the coloring agent, there may be exemplified food dyes such as beta-carotene, yellow iron sesquioxide, red iron sesquioxide, etc.

As the fluidizing agent or the lubricant, there may be exemplified sucrose esters of fatty acid, rapeseed oil powder, talc, light anhydrous silicic acid, calcium silicate, synthetic aluminum silicate, magnesium stearate, calcium stearate, sodium stearyl fumarate, etc.

As the excipient, there may be exemplified trehalose, mannitol, sorbitol, erythritol, lactitol, xylitol, maltitol, lactose, etc.

The intraorally rapidly disintegrable tablet according to the present invention may contain a lubricant inside the tablet. However, when a lubricant is contained inside the tablet, there occurs a phenomenon that wetting property of the tablet to water lowers as compared with a tablet containing no lubricant inside the tablet, due to the water-repelling property of the lubricant. Accordingly, it is preferred that no lubricant is contained inside the tablet.

Lubricant is a component which is usually added to a molding material for improving the fluidity of the molding material to be made into tablets and for preventing the problems upon tableting such as sticking, lamination and capping of the tablets and for preventing the creaking of a punch and a die in the tableting step, and its examples are stearic acid, metal stearates such as magnesium stearate and calcium stearate, sodium stearyl fumarate, sucrose fatty acid, talc, rapeseed fat powder, and the like. As a lubricant used for a nutritious food and a dietary supplement, it is preferred to use a lubricant mentioned in the Japanese Pharmacopoeia, official books for food additives, etc. for the sake of safety.

The intraorally rapidly disintegrable tablet according to the present invention is manufactured by compression molding of a mixture containing a saccharide selected from the group consisting of lactose, maltose, trehalose, lactitol, mannitol, erythritol, xylitol, sorbitol and maltitol, an amino acid and a disintegrating agent. The intraorally rapidly disintegrable tablet according to the present invention is also manufactured by a manner wherein a mixture containing an amino acid and a disintegrating agent is granulated with an aqueous solution of a binder and dried, and the resulting granules are subjected to compression molding.

Although there is no particular limitation for the granulation method, and the preparation by means of a wet granulation such as fluidized bed granulation, tumbling fluidized bed granulation, stirring granulation or extrusion granulation is preferred, dry granulation is employable. There is no particular limitation for particle size and specific surface area of those granules, and they can be determined discretionally so far as they have fluidity which does not deteriorate the tableting operation.

The aqueous solution of a binder may be manufactured by dissolving a binder in water such as pure water. Although there is no particular limitation for the dissolving temperature, it is preferably 40 to 100°C, more preferably 50 to 95°C and particularly preferably 60 to 90°C. Although there is no particular limitation for the granulating temperature, it is preferably 40 to 100°C, more preferably 40 to 95°C and particularly preferably 50 to 90°C. For example, when lactose is used as a binder, anhydrous lactose crystals are apt to be easily formed in the binder(lactose) bonding between amino acid particles and amino acid particles, between particle of a disintegrating agent and particle of a disintegrating agent, and between amino acid particles and particles of a disintegrating agent, which is likely to bring about a shape-holding ability against the physical force from outside.

In the intraorally rapidly disintegrable tablet of the present invention, which is prepared using the aqueous solution of lactose, there are crystals of anhydrous lactose, which can be detected by, for example, an X-ray diffraction method mentioned in, for example, *Yakuzaigaku*, 48(1), 1 (1988).

Although there is no particular limitation for the drying method, fluidized bed drying, ventilation drying, vacuum drying, etc, are exemplified. Although there is no particular limitation for the drying temperature, it is preferably 40 to 100°C, more preferably 50 to 95°C and particularly preferably 60 to 90°C.

In the present invention, there is no particular limitation for the method for the manufacture of tablets, and may be carried out by using, for example, a rotary tableting machine. As the tableting machine, a machine which is not equipped with a device for applying a lubricant to a punch and to a die may be used, however, it is preferred to use a machine equipped with a device for applying a lubricant to a punch and to a die. Compression molding may be carried out using a punch and a die to which a lubricant is applied or by using a punch and a die to which no lubricant is applied.

### Example 1

An amino acid mixture (5240 g) comprising 2620 g of glutamine, 1310 g of valine and 1310 g of isoleucine was mixed with 500 g of carboxymethylcellulose, and poured into a stirring granulator (trade name: Vertical Granulator VG-25; manufactured by Powrex Corporation). The mixture was mixed until it became homogeneous to give a mixture 3.

An aqueous solution of a binder prepared by dissolving 300 g of mannitol in 1500 g of pure water at 65°C was poured into a stirring granulator (trade name: Vertical Granulator VG-25; manufactured by Powrex Corporation) and kneaded for about 3 minutes.

Then, the kneaded product prepared as above was poured into an extrusion granulator (trade name: DGL-1; manufactured by Fuji Powder) equipped with a screen of diameter of 0.8 mm, and granulated.

The granulated product prepared as above was dried in a fluidized bed drier (trade name: FLO-5; manufactured by Freund Industrial Co., Ltd.) with warm air of about 50°C for 15 minutes to give granules.

The granules thus obtained were then made uniform using a wire net (No. 28) to give granules for tableting.

The obtained granules for tableting were subjected to compression molding by using a rotary tableting machine equipped with a plane punch of 12 mm diameter (trade name: AP-15; manufactured by Hata Tekkosho), in which a lubricant was applied to inner surface of the die, the upper surface of the lower punch and the lower surface of the upper punch prior to filling the above granules for tableting into a die, at a tableting pressure of 20 kN, and the intraorally rapidly disintegrable tablet having tablet weight of 540 mg was manufactured. In this Example, sucrose esters of fatty acid was used as a lubricant for the sake of safety on the ground that the intraorally rapidly disintegrable tablets to be manufactured would be used as a nutritious food or a dietary supplement.

Outline of the tablets manufactured is as follows.

Physical property of tablets:

| | |
|---|---|
| Tablet weight | 542 mg |
| Tablet diameter | 12 mm |
| Tablet thickness | 4.1 mm |
| Tablet hardness | 50 N |
| Intraorally disintegration time | about 29 seconds |

The resulting intraorally rapidly disintegrable tablets had a practical hardness showing no damage of the tablets during the distribution processes and packing steps after tableting, and were disintegrating quickly in the mouth cavity.

### Example 2

Into a stirring granulator (VG-25; manufactured by Powrex Corporation) were poured 2400 g of dextrin, 550 g of leucine, 450 g of isoleucine, 370 g of valine, 620 g of glutamine, 100 g of vitamin mix [comprising 1.10% by weight of vitamin A (1800 IU/g), 0.42% by weight of vitamin B₁, 0.60% by weight of vitamin B₆, 0.10% by weight of vitamin B₁₂, 0.56% by weight of nicotinic acid amide, 0.31% by weight of calcium pantothenate, 0.01% by weight of folic acid, 0.20% by weight of vitamin D₃ and 96.02% by weight of vitamin C] and 360 g of crospovidone, followed by mixing for about 3 minutes. Then, 400 g of 20% aqueous solution of trehalose was added thereto, followed by granulation, which was subjected to drying for 30 minutes with air of 80°C suction temperature using a fluidized bed drier (FLO-5; manufactured by Freund Industrial Co., Ltd.). The resulting dried granules were made uniform using a metal wire (No. 20) to give amino acid granules. To 2415 g of the amino acid granules were added 90 g of crospovidone and 1095 g of lactose, followed by mixing to prepare granules for tableting. Then, the obtained granules for tableting was subjected to compression molding by using a rotary tableting machine equipped with a device for applying a lubricant to which was attached a plane metal mold of 13 mm diameter (trade name: AP-15; manufactured by Hata Tekkosho), wherein magnesium stearate was applied to the inner surface of the die, upper surface of the lower punch and the lower surface of the upper punch prior to filling the above granules for tableting into the die, at a tableting pressure of 20 kN, and the intraorally rapidly disintegrable tablets having tablet weight of about 720 mg were manufactured.

Outline of the tablets manufactured is as follows.

Physical property of tablets:

| | |
|---|---|
| Tablet weight | 721 mg |
| Tablet diameter | 13 mm |
| Tablet thickness | 4.5 mm |
| Tablet hardness | 59 N |
| Intraorally Disintegration Time | about 32 seconds |

The resulting intraorally rapidly disintegrable tablets had a practical hardness showing no damage of the tablets during the distribution processes and packing steps after tableting and were disintegrating quickly in the mouth cavity.

### Example 3

Into a stirring granulator (VG-25; manufactured by Powrex Corporation) were poured 1240g of leucine, 900 g of isoleucine, 1180 g of valine, 700 g of arginine, 100 g of the vitamin mix which was the same one used in Example 2 and 180 g of crospovidone, followed by mixing for about 3 minutes. Then, 400 g of 20% aqueous solution of maltose was added thereto, followed by granulation, which was dried for 30 minutes with air of 80°C suction temperature using a fluidized bed drier (FLO-5; manufactured by Freund Industrial Co., Ltd.). The resulting dried granules were made uniform using a metal wire (No. 20) to give amino acid granules. To 2190 g of the amino acid granules were added 90 g of crospovidone, 1320 g of D-mannitol and 2 g of L-menthol, followed by mixing to prepare granules for tableting. Then, the obtained granules for tableting were subjected to compression molding by using a rotary tableting machine equipped with a device for applying a lubricant to which was attached a plane metal mold of 13 mm diameter (trade name: AP-15; manufactured by Hata Tekkosho), wherein magnesium stearate was applied to the inner surface of the die, upper surface of the lower punch and to the lower surface of the upper punch prior to filling the above granules for tableting into a die, at a tableting pressure of 20 kN, and the intraorally rapidly disintegrable tablets having tablet weight of about 720 mg were manufactured.

Outline of the tablets manufactured is as follows.

Physical property of tablets:

| | |
|---|---|
| Tablet weight | 720 mg |
| Tablet diameter | 13 mm |
| Tablet thickness | 4.5 mm |
| Tablet hardness | 52 N |
| Intraorally disintegration Time | about 27 seconds |

The resulting intraorally rapidly disintegrable tablets had a practical hardness showing no damage of the tablets during the distribution processes and packing steps after tableting and were disintegrating quickly in the mouth cavity.

### Example 4

Into a stirring granulator (trade name: Vertical Granulator VG-25; manufactured by Powrex Corporation) were poured 5000 g of L-glutamine (manufactured by Kyowa Hakko Kogyo Co., Ltd. ) and 270 g of carboxymethylcellulose calcium (CMC-Ca), followed by mixing for about 3 minutes. Granulation was carried out by a manner wherein 800 g of 20% aqueous solution of lactose was added to the mixture and kneaded by a stirring granulator (trade name: Vertical Granulator VG-25; manufactured by Powrex Corporation), and the resulting product was poured into an extrusion granulator (trade name: DGL-1; manufactured by Fuji Powder) equipped with a screen having a diameter of 0.8 mm for granulation. After that, the product thus obtained was dried for 15 minutes with hot air of about 80°C using a fluidized bed drier (FLO-5; manufactured by Freund Industrial Co., Ltd.) to give granules.

The resulting dried granules were made uniform using a metal wire (No. 20) to give granules for tableting.

Then, the obtained granules for tableting was subjected to compression molding by using a rotary tableting machine equipped with a device for applying sucrose esters of fatty acid on the punch surface and the die wall, wherein sucrose esters of fatty acid was applied to the inner surface of the die, the upper surface of the lower punch and to the lower surface of the upper punch prior to filling the granules for tableting into a die, at a tableting pressure of 20 kN, and the intraorally rapidly disintegrable tablets was manufactured.

Outline of the tablets manufactured is as follows.

Physical property of tablets:

| | |
|---|---|
| Tablet weight | 544 mg |
| Tablet diameter | 12 mm |
| Tablet thickness | 4.1 mm |
| Tablet hardness | about 40 N |
| Intraorally disintegration time | about 30 seconds |

The resulting intraorally rapidly disintegrable tablets had a practical hardness showing no damage of the tablets during the distribution processes and packing steps after tableting and were disintegrating quickly in the mouth cavity.

### Example 5

Into a stirring granulator (trade name: Vertical Granulator VG-25; manufactured by Powrex Corporation) were poured 5000 g of L-glutamine (manufactured by Kyowa Hakko Kogyo Co., Ltd.), 55 g of orange flavor and 270 g of crospovidone, followed by kneading for about 3 minutes. Granulation was carried out by a manner wherein 800 g of 20% aqueous solution of lactose was added to the mixture and kneaded by a stirring granulator (trade name: Vertical Granulator VG-25; manufactured by Powrex Corporation), and the resulting product was poured into an extrusion granulator (trade name: DGL-1; manufactured by Fuji Powder) equipped with a screen having a diameter of 0.8 mm for granulation. After that, the product thus obtained was dried for 15 minutes with hot air of about 80°C using a fluidized bed drier (FLO-5; manufactured by Freund Industrial Co., Ltd.) to give granules.

The resulting granules were made uniform using a metal wire (No. 20) to give granules for tableting.

Then, the obtained granules for tableting was subjected to compression molding by using a rotary tableting machine equipped with a device for applying sucrose esters of fatty acid on the punch surface and the die wall, wherein sucrose esters of fatty acid was applied to the inner surface of the die, the upper surface of the lower punch and to the lower surface of the upper punch prior to filling the above granules for tableting into a die, at a tableting pressure of 20 kN, and the intraorally rapidly disintegrable tablets was manufactured.

Outline of the tablets manufactured is as follows.

Physical property of tablets:

| | |
|---|---|
| Tablet weight | 542 mg |
| Tablet diameter | 12 mm |
| Tablet thickness | 4.0 mm |
| Tablet hardness | 59 N |
| Intraorally disintegration time | about 22 seconds |

The resulting intraorally rapidly disintegrable tablets had a practical hardness showing no damage of the tablets during the distribution processes and packing steps after tableting and were disintegrating quickly in the mouth cavity.

### Example 6

The same procedure as in Example 4 was carried out except that a component for a binder was substituted with trehalose, whereby intraorally rapidly disintegrable tablets were prepared.

Outline of the tablets manufactured is as follows.

Physical property of tablets:

| | |
|---|---|
| Tablet weight | 546 mg |
| Tablet diameter | 12 mm |
| Tablet thickness | 4.0 mm |
| Tablet hardness | 43 N |
| Intraorally disintegration time | about 32 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Example 7

The same procedure as in Example 4 was carried out except that a component for a binder was substituted with maltose, whereby intraorally rapidly disintegrable tablets were prepared.

Outline of the tablets manufactured is as follows.

Physical property of tablets:

| | |
|---|---|
| Tablet weight | 545 mg |
| Tablet diameter | 12 mm |
| Tablet thickness | 4.1 mm |
| Tablet hardness | 47 N |
| Intraorally disintegration time | about 29 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Example 8

The same procedure as in Example 4 was carried out except that a component for a binder was substituted with sorbitol, whereby intraorally rapidly disintegrable tablets were prepared.

Outline of the tablets manufactured is as follows.

Physical property of tablets:

| | |
|---|---|
| Tablet weight | 544 mg |
| Tablet diameter | 12 mm |
| Tablet thickness | 4.0 mm |
| Tablet hardness | 48 N |
| Intraorally disintegration time | about 28 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Example 9

The same procedure as in Example 4 was carried out except that a component for a binder was substituted with maltitol, whereby intraorally rapidly disintegrable tablets were prepared.

Outline of the tablets manufactured is as follows.

Physical property of tablets:

| | |
|---|---|
| Tablet weight | 543 mg |
| Tablet diameter | 12 mm |
| Tablet thickness | 4.1 mm |
| Tablet hardness | 49 N |
| Intraorally disintegration time | about 30 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Example 10

The same procedure as in Example 2 was carried out except that a binder was substituted with pure water, whereby intraorally rapidly disintegrable tablets were prepared.

Outline of the tablets manufactured is as follows.

Physical property of tablets:

| | |
|---|---|
| Tablet weight | 720 mg |
| Tablet diameter | 13 mm |
| Tablet thickness | 4.50 mm |
| Tablet hardness | 40 N |
| Intraorally disintegration time | about 25 seconds |

It was found that, even when pure water was used as a binder, it was possible to prepare the intraorally rapidly disintegrable tablets having a practical hardness and disintegrating quickly in the mouth cavity.

### Example 11

The same procedure as in Example 4 was carried out except that a component for a binder was substituted with erythritol, whereby intraorally rapidly disintegrable tablets were prepared.

Outline of the tablets manufactured is as follows.

Physical property of tablets:

| | |
|---|---|
| Tablet weight | 545 mg |
| Tablet diameter | 12 mm |
| Tablet thickness | 4.0 mm |
| Tablet hardness | 47 N |
| Intraorally disintegration time | about 29 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Example 12

The same procedure as in Example 4 was carried out except that carboxymethylcellulose calcium (CMC-Ca) as a disintegrating agent was substituted with proline, whereby intraorally rapidly disintegrable tablets were prepared.

Outline of the tablets manufactured is as follows.

Physical property of tablets:

| | |
|---|---|
| Tablet weight | 545 mg |
| Tablet diameter | 12 mm |
| Tablet thickness | 4.1 mm |
| Tablet hardness | 42 N |
| Intraorally disintegration time | about 29 seconds |

As a result, it was found that there were prepared the intraorally rapidly disintegrable tablets which were nearly the same as those in Example 4 having a practical hardness showing no damage of the tablets during the distribution processes and packing steps after tableting and were disintegrating quickly in the mouth cavity.

### Industrial Applicability

There is provided, according to the present invention, an intraorally rapidly disintegrable tablet as a preparation containing an amino acid as a principal agent, which disintegrates quickly upon contact to saliva in the mouth cavity and can be taken without water, and a method for manufacturing the same.

There are also provided, according to the present invention, an intraorally rapidly disintegrable tablet in which various kinds of amino acids are uniformly dispersed, and a method for manufacturing the same.

There are also provided, according to the present invention, an intraorally rapidly disintegrable tablet as a preparation containing an amino acid, which is excellent in stability, as a principal agent, and a method for manufacturing the same.

There are also provided, according to the present invention, an intraorally rapidly disintegrable tablet as a preparation containing an amino acid as a principal agent which quickly disintegrates in the mouth cavity, and a method for manufacturing the same.

There are also provided, according to the present invention, an intraorally rapidly disintegrable tablet as a preparation containing an amino acid as a principal agent which hardly cracks even by a physical force caused during storage and transportation, and a method for manufacturing the same.

There are also provided, according to the present invention, an intraorally rapidly disintegrable tablet containing an amino acid which contains no effervescent component and hardly stimulates the mouth cavity, and a method for manufacturing the same.

## Claims

1. An intraorally rapidly disintegrable tablet which comprises a saccharide selected from the group consisting of lactose, maltose, trehalose, sorbitol, lactitol, mannitol, erythritol, xylitol and maltitol, one member selected from the group consisting of an amino acid and an amino acid derivative and a disintegrating agent.

2. An intraorally rapidly disintegrable tablet which is manufactured by granulating a mixture containing one member selected from the group consisting of an amino acid and an amino acid derivative and a disintegrating agent with an aqueous solution of a binder to prepare granules, drying the granules, and subjecting the resulting granules to compression molding.

3. An intraorally rapidly disintegrable tablet which is manufactured by granulating a mixture containing one member selected from the group consisting of an amino acid and an amino acid derivative and a disintegrating agent with an aqueous solution of a binder to prepare granules, drying the granules, and subjecting a molding material comprising a mixture of the resulting granule and an additive to compression molding..

4. The intraorally rapidly disintegrable tablet according to claim 2 or claim 3, wherein the aqueous solution of a binder is an aqueous solution of lactose.

5. The intraorally rapidly disintegrable tablet according to claim 2 or claim 3, wherein the aqueous solution of a binder is an aqueous solution selected from the group consisting of an aqueous solution of maltose, an aqueous solution of trehalose, an aqueous solution of sorbitol, an aqueous solution of erythritol, an aqueous solution of xylitol, an aqueous solution of lactitol, an aqueous solution of mannitol and an aqueous solution of maltitol.

6. The intraorally rapidly disintegrable tablet according to any of claims 3 to 5, wherein the additive contains an excipient selected from the group consisting of trehalose, mannitol, sorbitol, erythritol, lactitol, xylitol, maltitol and lactose.

7. The intraorally rapidly disintegrable tablet according to any of claims 1 to 6, wherein the tablet is an externally lubricated tablet.

8. The intraorally rapidly disintegrable tablet according to any of claims 1 to 7, which is a pharmaceutical preparation, a nutritious food or a dietary supplement.

9. A method for the manufacture of an intraorally rapidly disintegrable tablet comprising the steps of:
preparing a mixture containing a saccharide compound selected from the group consisting of lactose, maltose, trehalose, sorbitol, erythritol, lactitol, mannitol, xylitol and maltitol, an amino acid and a disintegrating agent, and
subjecting the mixture to compression molding.

10. A method for the manufacture of an intraorally rapidly disintegrable tablet comprising the steps of:
preparing a mixture containing one member selected from the group consisting of an amino acid and an amino acid derivative and a disintegrating agent;
mixing the mixture with an aqueous solution of a binder to prepare granules;
drying the granules; and
subjecting the granules to compression molding.

11. A method for the manufacture of an intraorally rapidly disintegrable tablet comprising the steps of:
preparing a mixture containing one member selected from the group consisting of an amino acid and an amino acid derivative and a disintegrating agent,
mixing the mixture with an aqueous solution of a binder to prepare granules;
drying the gralunes;
mixing the granules with an additive to prepare a molding material; and
subjecting the molding material to compression molding.

12. The method for the manufacture of an intraorally rapidly disintegrable tablet according to claim 10 or claim 11, wherein the aqueous solution of a binder is an aqueous solution of lactose.

13. The method for the manufacture of an intraorally rapidly disintegrable tablet according to claim 10 or claim 11, wherein the aqueous solution of a binder is an aqueous solution selected from the group consisting of an aqueous solution of maltose, an aqueous solution of trehalose, an aqueous solution of erythritol, an aqueous solution of xylitol, an aqueous solution of lactitol, an aqueous solution of mannitol, an aqueous solution of sorbitol and an aqueous solution of maltitol.

14. The method for the manufacture of an intraorally rapidly disintegrable tablet according to any of claims 11 to 13, wherein the additive contains an excipient selected from the group consisting of trehalose, mannitol, erythritol, lactitol, maltitol, sorbitol, xylitol and lactose.

15. The method for the manufacture of an intraorally rapidly disintegrable tablet according to any of claims 9 to 14, wherein the step of subjecting the molding material to compression molding is **characterized by** using a punch and a die to which a lubricant is applied.

16. The method for the manufacture of an intraorally rapidly disintegrable tablet according to any of claims 9 to 15, wherein the intraorally rapidly disintegrable tablet manufactured is a pharmaceutical preparation, a nutritious food or a dietary supplement.

17. An intraorally rapidly disintegrable tablet which contains a saccharide selected from the group consisting of lactose, maltose, trehalose, sorbitol and maltitol, one member selected from the group consisting of an amino acid and an amino acid derivative and a disintegrating agent.

18. An intraorally rapidly disintegrable tablet which is manufactured by granulating a mixture containing one member selected from the group consisting of an amino acid and an amino acid derivative and a disintegrating agent with an aqueous solution of a binder to prepare granules, drying the granules, and subjecting the resulting granules to compression molding.

19. An intraorally rapidly disintegrable tablet which is manufactured by granulating a mixture containing one member selected from the group consisting of an amino acid and an amino acid derivative and a disintegrating agent with an aqueous solution of a binder to prepare granules, drying the granules, and subjecting a molding material comprising a mixture of the resulting granule and an additive to compression molding.

20. The intraorally rapidly disintegrable tablet according to claim 18 or claim 19, wherein the aqueous solution of a binder is an aqueous solution of lactose.

21. The intraorally rapidly disintegrable tablet according to claim 18 or claim 19, wherein the aqueous solution of a binder is an aqueous solution selected from the group consisting of an aqueous solution of maltose, an aqueous solution of trehalose, an aqueous solution of sorbitol and an aqueous solution of maltitol.

22. The intraorally rapidly disintegrable tablet according to any of claims 19 to 21, wherein the additive contains an excipient selected from the group consisting of trehalose, mannitol and lactose.

23. The intraorally rapidly disintegrable tablet according to any of claims 17 to 22, wherein the tablet is an externally lubricated tablet.

24. The intraorally rapidly disintegrable tablet according to any of claims 17 to 23, which is a pharmaceutical preparation, a nutritious food or a dietary supplement.

25. A method for the manufacture of an intraorally rapidly disintegrable tablet comprising the steps of:
preparing a mixture containing a saccharide compound selected from the group consisting of lactose, maltose, trehalose, sorbitol and maltitol, one member selected from the group consisting of an amino acid and an amino acid derivative and a disintegrating agent; and
subjecting the mixture to compression molding.

26. A method for the manufacture of an intraorally rapidly disintegrable tablet comprising the steps of:
preparing a mixture containing one member selected from the group consisting of an amino acid and an amino acid derivative and a disintegrating agent;
mixing the mixture with an aqueous solution of a binder to prepare granules;
drying the granules; and
subjecting the granules to compression molding.

27. A method for the manufacture of an intraorally rapidly disintegrable tablet comprising the steps of:
preparing a mixture containing one member selected from the group consisting of an amino acid and an amino acid derivative and a disintegrating agent;
mixing the mixture with an aqueous solution of a binder to prepare granules
drying the granules;
mixing the granules with an additive to prepare a molding material; and
subjecting the molding material to compression molding.

28. The method for the manufacture of an intraorally rapidly disintegrable tablet according to claim 26 or claim 27, wherein the aqueous solution of a binder is an aqueous solution of lactose.

29. The method for the manufacture of an intraorally rapidly disintegrable tablet according to claim 26 or claim 27, wherein the aqueous solution of a binder is an aqueous solution selected from the group consisting of an aqueous solution of maltose, an aqueous solution of trehalose, an aqueous solution of sorbitol and an aqueous solution of maltitol.

30. The method for the manufacture of an intraorally rapidly disintegrable tablet according to any of claims 27 to 29, wherein the additive contains an excipient selected from the group consisting of trehalose, mannitol and lactose.

31. The method for the manufacture of an intraorally rapidly disintegrable tablet according to any of claims 25 to 30, wherein the step of subjecting the molding material to compression molding is **characterized in** using a punch and a die to which a lubricant is applied.

32. The method for the manufacture of an intraorally rapidly disintegrable tablet according to any of claims 25 to 31, wherein the intraorally rapidly disintegrable tablet manufactured is a pharmaceutical preparation, a nutritious food or a dietary supplement.
